# EUROPEAN PATENT APPLICATION

(11) **EP 3 002 027 A1**
(43) Date of publication of application: **06.04.2016**
(21) Application number: 14810769.1
(22) Date of filing: 13.06.2014
(51) Int. Cl.: A61M 16/20

(54) **PNEUMATIC ELECTRICAL SWITCH AND MEDICAL EQUIPMENT PROVIDED WITH SAME**

(30) Priority: 14.06.2013 CN 201310237826
(71) Applicant: Beijing Aeonmed Co., Ltd., Beijing 100070 (CN)
(72) Inventor: BI, Huimin, Beijing 100070 (CN)
(74) Representative: Hoarton, Lloyd Douglas Charles
(86) International application number: PCT/CN2014/079856
(87) International publication number: WO 2014/198235

(57) **Abstract**

The invention discloses a pneumatic-electric linked switch and a medical device with the pneumatic-electric linked switch. The pneumatic-electric linked switch comprises a pneumatic valve assembly, a connecting piece, a transmission piece and a driving piece, wherein the pneumatic valve assembly comprises a valve body and a valve element, an air passage is arranged in the valve body, the valve element is arranged on the valve body in the mode that the valve element can move between a connection position where the valve element is communicated with the air passage and a disconnection position where the valve element is disconnected with the air passage, the connecting piece is arranged on the pneumatic valve assembly, the transmission piece is arranged on the connecting piece in the mode that the transmission piece can move between an opening position and a closing position and is matched with the valve element, the valve element is located at the connection position when the transmission piece is located at the opening position, the valve element is located at the disconnection position when the transmission piece is located at the closing position, and the driving piece is connected with the transmission piece so as to drive the driving piece to move between the opening position and the closing position. The pneumatic-electric linked switch has the advantages of being simple in structure, convenient to disassemble and assemble, low in manufacturing cost and the like.

## Description

The patent application claims priority of Chinese Patent Application No. 201310237826.5, entitled Pneumatic Electrical Switch and Medical Equipment Provided with Same, filed on June 14, 2013, by Beijing Aeonmed Co. Ltd., the disclosure of which is incorporated herein by reference in its entirety.

### Technical Field

The invention relates to a pneumatic electrical switch and medical equipment provided with same.

### Background Art

A pneumatic electrical switch is used for accurately integrating an air circuit with an electric circuit. According to the existing pneumatic electrical switch, a switch knob is connected to a pneumatic valve component through a complicated cam installation member. Therefore, the existing pneumatic electrical switch has the defects of complicated structure, higher processing cost and inconvenient dismounting.

### Summary of the Invention

The purpose of this invention is to at least solve one of the technical problems in the prior art. Therefore, one object of the invention is to provide a pneumatic electrical switch.

Another object of the invention is to provide a medical equipment provided with the pneumatic electrical switch.

In order to realizing the above objects, according to the embodiment of a first aspect of the invention, there is provided a pneumatic electrical switch. The pneumatic electrical switch comprises a pneumatic valve component including a valve being internally provided with an air passage and a valve core being movably installed on the valve body between a connection position that connects to the air passage and a disconnection position that disconnects to the air passage, a connecting member being installed on the pneumatic valve component, a transmission member being movably installed on the connecting member between an opening position and a closing position and matched with the valve core; when the transmission member is located at the opening position, the valve core is located at the connection position, and when the transmission member is located at the closing position, the valve core is located at the disconnection position; a driving member being connected to the transmission member in order to drive the transmission member to move between the opening position and the closing position.

The pneumatic electrical switch according to the embodiment of the invention sets the connecting member and the transmission member being movably installed on the connecting member between an opening position and a closing position and matched with the valve core, which is aimed at realizing the connection between the pneumatic valve component and the driving member in order to simplify the structure of the pneumatic electrical switch to install and dismount it conveniently. In addition, it is beneficial to reduce the processing difficulty and the processing cost of the pneumatic electrical switch because of the simple structure. Therefore, the pneumatic electrical switch according to the embodiment of the invention has the advantages of simple structure, convenient dismantling, low manufacturing cost etc.

In addition, the pneumatic electrical switch according to the embodiment of the invention may also have the additional technical features as follows.

According to one embodiment of the invention, there is a plurality of air passages, a plurality of valve cores being correspondingly matched with the plurality of air passages, and a plurality of transmission members being installed and correspondingly matched with the plurality of valve cores respectively. Thus, it is possible to realize the connection and disconnection of the multiple air passages to make the application scope of the pneumatic electrical switch be wider.

According to one embodiment of the invention, the connecting member comprises a connecting member body installing a mounting hole penetrating being movably matched with the transmission member through the connecting member body, a first connecting arm being installed on the connecting member body and connected to the pneumatic valve component, and a second connecting arm being installed on the connecting member body and connected to the pneumatic valve component, wherein the second connecting arm is separated from the first connecting arm. The connecting member may be connected to the pneumatic valve component more steadily by setting the first connecting arm and the second connecting arm.

According to one embodiment of the invention, the connecting member body, the first connecting arm and the second connecting arm are formed integrally. Therefore, not only the structural strength of the connecting member can be improved, but also the processing cost and processing difficulty of the connecting member can be reduced.

According to one embodiment of the invention, the first connecting arm is connected to the first flank of the valve body; the second connecting arm is connected to the second flank of the valve body. Thereof the first flank is opposite to the second flank. Therefore, the connecting member can be connected to the pneumatic valve component more steadily.

According to one embodiment of the invention, the connecting member body sets a through hole penetrating through the connecting member body along the thickness direction of the connecting member body in order to reduce the weight of the connecting member body and the processing cost.

According to one embodiment of the invention, the driving member comprises a fixing portion being installed on the connecting member, a driving member body being rotatable installed on the fixing portion and a groove being installed at the end surface of the driving member body; one side wall of the groove being installed in an inclined way, and thereof the driving member being contacted with the bottom wall of the groove at the closing position, and the driving member being contacted with the end face of the driving member body at the opening position, and a holding portion being installed on the driving member body.

According to one embodiment of the invention, a clamping trough being installed inside the fixing portion and a part of the connecting member is matched with the clamping trough in order to make the connection between the fixing portion and the connecting member be more firmly.

According to the embodiment of a second aspect of the invention, there is provided a medical equipment. The medical equipment comprises the pneumatic electrical switch according to the first aspect of the invention. Therefore, the medical equipment has the advantages of simple structure, convenient installation and dismounting, low fabrication cost etc.

According to one embodiment of the invention, the medical equipment is a ventilator or an anesthesia machine.

The invention will present the additional aspects and advantages in the following description, and the partial additional aspects and advantages could become obvious in the following description or via the practice of the invention.

### Brief Description of the Drawings

The above and/or additional aspects and advantages of the invention will become obvious and easy to understand from the description of embodiments with reference to the following drawings, wherein:
Fig. 1 is a schematic structural diagram of a pneumatic electrical switch according to embodiments of the invention;
Fig. 2 is a schematic structural diagram of a pneumatic electrical switch in a different visual angle according to the embodiments of the invention;
Fig. 3 is a partial section view of a connecting member of the pneumatic electrical switch according to the embodiments of the invention;
Fig. 4 is a schematic structural diagram of the pneumatic valve component of the pneumatic electrical switch according to the embodiments of the invention;
Fig. 5 is a partial section view of the pneumatic valve component of the pneumatic electrical switch according to the embodiments of the invention; and
Fig. 6 is a partial section view of a valve body of the pneumatic electrical switch according to the embodiments of the invention.

### Detailed Description of the Preferred Embodiments

Hereinafter, embodiments of the invention are described in detail, examples of which are shown in the drawings, wherein the same or similar labels represent the same or similar component or the component of the same or similar function. The following embodiments described with reference to the drawings are exemplary, which are aimed at explaining the invention rather than restricting the invention,

In the description of the invention, the technical words are "center", "width", "thickness", "upper", "lower", "front", "back", "left", "right", "up-down", "horizontal", "top", "bottom", "inner", "outer" et al. these words represent orientation or position relationship shown based on the drawings in order to describe the invention and simplify the description and not to indicate or imply that the indicated device or component must have a specific orientation or constructed and operated in a specific orientation. Therefore, it is not to be understood as a restriction for the invention. Also the technical words "first", "second" are only be used for a descriptive purpose, and are not to be understood to indicate or imply the relative importance or imply the number of the indicated technical features. Therefore, features defined with "first", "second" may include one or more of the features explicitly or implicitly. In the description of the invention, "multiple" means two or more than two, unless specifically defined otherwise.

In the invention, the technical words "install", "be linked to", "connect to", "fix" et al. should be understood in generalization, unless specifically defined otherwise. For example, these technical words are described as fixed joint or removable connection or the integration of the connection; or mechanical joint or electrical connection; or direct connection or indirect connection via the middle medium or internal connection between the two component. The skilled persons in the art can understand the specific meaning about these technical words in the invention according to the specific circumstance.

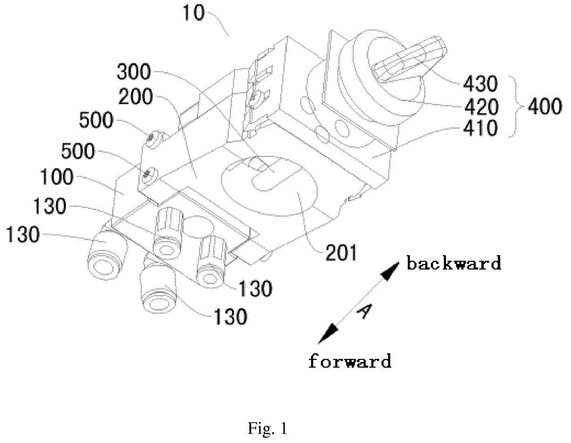

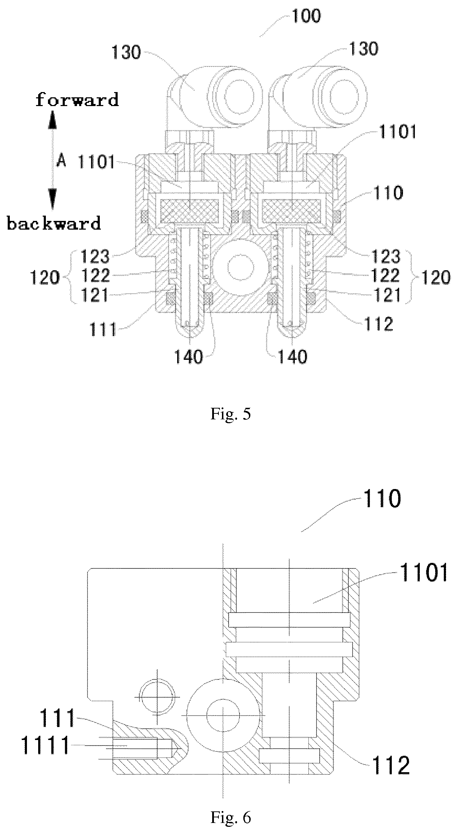

A pneumatic electrical switch 10 according to the embodiment of the invention is described hereinafter with reference to Fig. 1 to Fig. 6. As shown in Fig. 1 to Fig. 6, the pneumatic electrical switch 10 comprises a pneumatic valve component 100, a connecting member 200, a transmission member 300 and a driving member 400.

The pneumatic valve component 100 comprises a valve body 110 being internally provided with an air passage 1101 and a valve core 120 being movably installed on the valve body 110 between a connection position that connects to the air passage 1101 and a disconnection position that disconnects to the air passage 1101. The connecting member 200 is installed on the pneumatic valve component 100. The transmission member 300 is movably installed on the connecting member 200 between an opening position and a closing position and matched with the valve core 120; thereof when the transmission member 300 is located at the opening position, the valve core 120 is located at the connection position, and when the transmission member 300 is located at the closing position, the valve core 120 is located at the disconnection position. The driving member 400 is connected to the transmission member in order to drive the transmission member 300 to move between the opening position and the closing position.

The pneumatic electrical switch 10 is used for integrating an air circuit with an electric circuit. When the transmission member 300 is installed at the closing position, the valve core 120 is located at the disconnection position with the air passage 1101 being located at the disconnection position and then the pneumatic electrical switch 10 is turned off. In the application, the motion direction of the transmission member 300 when connecting the air passage 1101 is taken as the forward direction, and the motion direction of the transmission member 300 when disconnecting the air passage 1101 is taken the backward direction, and then the forward direction and the backward direction are as shown by arrow A in Fig. 1, Fig. 2 and Fig. 5.When the pneumatic electrical switch 10 needs to be turned on, the driving member 400 drive the transmission member 300 to move to the opening position from the closing position, and the transmission member 30 drives the valve core 120 to move to the connection position from the disconnection position meanwhile, i.e., the transmission member 300 drives the valve core 120 to move forward, so that the air passage 1101 is connected. When the pneumatic electrical switch 10 needs to be turned off, the motion directions of the transmission member 300 and the valve core 120 are opposite to the motion direction when turning on the pneumatic electrical switch 10, which will be not described in details hereinafter.

Therefore, the pneumatic electrical switch 10 according to the embodiment of the invention sets the connecting member 200 and the transmission member 300 being movably installed on the connecting member between an opening position and a closing position and matched with the valve core 120, which is aimed at realizing the connection between the pneumatic valve component 100 and the driving member 400 in order to simplify the structure of the pneumatic electrical switch 10 to install and dismount it conveniently. In addition, it is beneficial to reduce the processing difficulty and the processing cost of the pneumatic electrical switch 10 because of the simple structure. Therefore, the pneumatic electrical switch 10 according to the embodiment of the invention has the advantages of simple structure, convenient dismantling, low manufacturing cost etc.

As shown in Fig. 1 to Fig. 3, in some embodiments of the invention, the connecting member 200 may include a connecting member body 210, a first connecting arm 220 and a second connecting arm 230. The connecting member body 210 may be provided with a mounting hole 211 penetrating through the connecting member body 210 (specifically, the mounting hole 211 penetrates through the connecting member body 210 along the direction of front and back), and the transmission member 300 is movably matched with the mounting hole 211 in order to move between the opening position and the closing position. The first connecting arm 220 may be installed on the connecting member body 210 and connected to the pneumatic valve component 100, the second connecting arm 230 may be installed on the connecting member body 210 and connected to the pneumatic valve component 100, and the second connecting arm 230 is separated from the first connecting arm 220.The connecting member 200 may be connected to the pneumatic valve component 100 more steadily by setting the first connecting arm 220 and the second connecting arm 230.

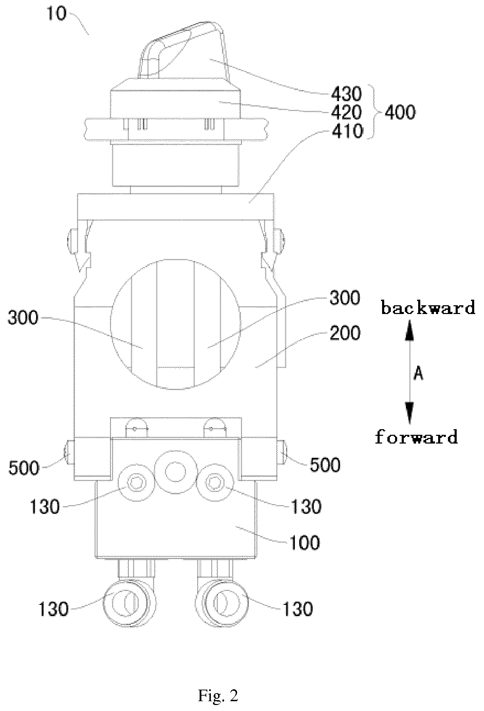

Specifically, as shown in Fig. 1 and Fig. 2, the first connecting arm 220 may be connected with the first flank 111 of the valve body 110, the second connecting arm 230 is connected with the second flank 112 of the valve body 110, and the first flank 111 is opposite to the second flank 112.In other words, the valve body 110 may be clamped between the first connecting arm 220 and the second connecting arm 230,thereby enabling the connecting member 200 to connect with the pneumatic valve component 100 more steadily.

As shown in Fig. 1, Fig. 2 and Fig. 6, advantageously, the first flank 111 of the valve body 110 may install a first screw hole 1111, the first connecting arm 220 may install a first through hole 2201 penetrating through the first connecting arm 220, a screw 500 may be penetrated through the first through hole 2201 and threaded with the first screw hole 1111 in order to make the first connecting arm 220 be connected to the valve body 110 together more easily and steadily. The second side 112 of the valve body 110 may install a second screw hole 1121, the second connecting arm 230 may install a second through hole 2301 penetrating through the second connecting arm 230, the screw 500 may penetrate through the second through hole 2301 and threaded with the second screw hole 1121in order to make the second connecting arm 230 be connected with the valve body 110 together more easily and steadily. Moreover, the pneumatic electrical switch 10 may further be dismounted more easily so as to repair and replace parts.

A plurality of screws 500 may be arranged, and a plurality of first screw holes 1111, a plurality of first through holes 2201, a plurality of second screw holes 1121 and a plurality of second through holes 2301 may be arranged. Therefore, the pneumatic valve component 100 may be connected to the connecting member 200 in more firmly.

The connecting member body 210, the first connecting arm 220 and the second connecting arm 230 may be formed integrally. Therefore, not only the structural strength of the connecting member 200 can be improved, but also the processing cost an processing difficulty of the connecting member 200 can be reduced.

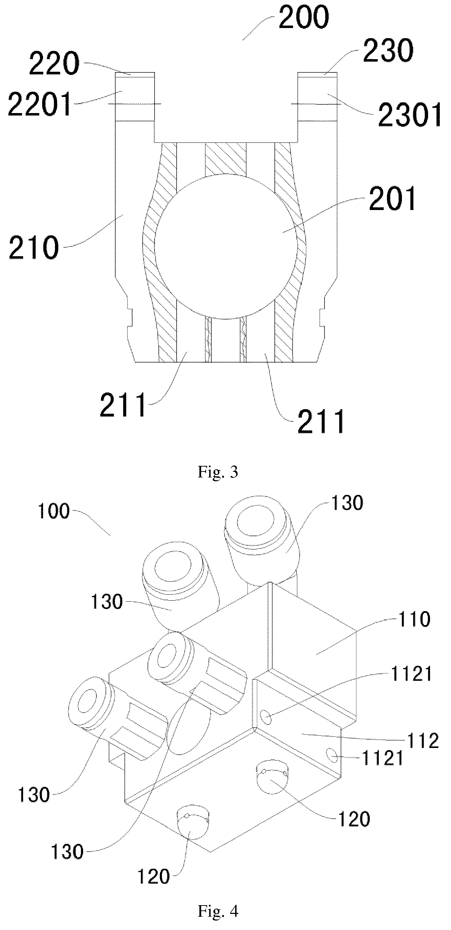

As shown in Fig. 3, in one embodiment of the invention, the connecting member body 210 may install a through hole 201 penetrating through the connecting member body 210 along the thickness direction of the connecting member body 210in order to reduce the weight of the connecting member body 210 and the fabrication cost.

In a specific example of the invention, as shown in Fig. 1 and Fig. 2, the driving member 400 may include a fixing portion 410, a driving member body 420 and a holding portion 430. The fixing portion 410 may be installed on the connecting member 200. The driving member body 420 may be rotatable installed on the fixing portion 410, the end face of the driving member body 420 may be installed a groove, and one side wall of the groove is installed in an inclined way. The transmission member 300 may be contacted with the bottom wall of the groove in the closing position, and the transmission member 300 may be contacted with the end face of the driving member body 420 in the opening position. The holding portion 430 may be arranged on the driving member body 420, thereby facilitating a user to rotate the driving member 400.

When the air passage 1101 needs to be connected, adopting the method to rotate the holding portion 430 is to drive and rotate the driving member body 420. When the driving member body 420 rotates to the lower edge of one side wall of the groove and is contacted with the transmission member 300, the driving member body 420 continues to be rotated, one side wall of the groove may drive the transmission piece 300 to move from the closing position to the opening position so that the transmission member 300 may drive the valve core 120 to move to the connection position from the disconnection position so as to connect to the air passage 1101. When the transmission member 300 is contacted with the end face of the driving member body 420, the transmission member 300 is located at the opening position and does not move any more. The process of disconnecting the air passage 1101 is reverse to the process of connecting the air passage, which will not be described in details here.

Advantageously, the fixing portion 410 may be internally provided with a clamping trough, and a part of the connecting member 200 may be fit in the clamping trough. In other words, the clamping trough may be clamped on the connecting member 200. Therefore, the fixing portion 410 may be connected to the connecting member 200 more firmly.

To be specific, a part of the connecting member body 210 may be fit in the clamping trough. A fastener (such as a screw) may penetrate through the fixing portion 410 (namely penetrating through the wall of the clamping trough) and be threaded in the connecting member body 210.

As shown in Fig. 1 to Fig. 6, in some embodiments of the invention, a plurality of air passages 1101 may be arranged, a plurality of valve cores 120 may be arranged, and the plurality of valve cores 120 may be correspondingly matched with the plurality of air passages 1101 respectively. In other words, the number of the valve cores 120 may be equal to the number of the air passages 1101, and one valve core 120 may be matched with one air passage1101. A plurality of transmission members 300 may be arranged and the plurality of transmission members 300 may be correspondingly matched with the plurality of valve cores 120 respectively. In other words, the number of the transmission members 300 may be equal to the number of the valve cores 120, and one transmission member 300 may be matched with one valve core 120. Therefore, the connection and disconnection of the multiple air passages 1101 are implemented, so that the application scope of the pneumatic electrical switch 10 is wider.

Those skilled in the art may understand that the valve core 120 may include a valve core body 121, an elastic element 122 and a sealing gasket 123. The valve body 110 may be provided with a quick plug 130 communicated with an outside air circuit. A sealing element 140 for sealing may be installed between the valve core 120 and the valve body 110. Since the above-mentioned structure and operating principle of the pneumatic valve component 100 are known and is not correlated to the inventive points of the invention, the detailed description will not be presented out any more.

The invention further provides a medical equipment. The medical equipment according to the embodiment of the invention includes the pneumatic electrical switch 10 according to the above-mentioned embodiment.

Via setting the pneumatic electrical switch 10 according to the above-mentioned embodiment, the medical equipment according to the embodiment of the invention has the advantages of simple structure, convenient dismantling, low manufacturing cost, etc.

Optionally, the medical equipment may be a ventilator or an anesthesia machine.

In the description of the specification, references to the technical words "an embodiment", "some embodiments", "an example", "a specific example" or "some examples" et al. mean that specific features, structures, materials described in combination with the embodiment or example are embodied in at least one embodiment or example of the invention. In the specification, the schematic expressions of these technical words do not necessarily refer to the same embodiment or example. Furthermore, the described specific features, structures, materials or characteristics may be combined within any of one or more embodiments or examples in a suitable manner.

Although embodiments of the invention have been illustrated and described in the above, it is to be understood that the above mentioned embodiments are exemplary rather than the limitation of the invention. Those skilled in the art may make alterations, substitution and modifications to the above mentioned embodiments within the scope of the invention and without departing from the principle and gist of the invention.

## Claims

1. A pneumatic electrical switch, wherein comprising:
a pneumatic valve component including a valve being internally provided with an air passage and a valve core being movably installed on the valve body between a connection position that connects to the air passage and a disconnection position that disconnects to the air passage,
a connecting member being installed on the pneumatic valve component,
a transmission member being movably installed on the connecting member between an opening position and a closing position and matched with the valve core; when the transmission member is located at the opening position, the valve core is located at the connection position, and when the transmission member is located at the closing position, the valve core is located at the disconnection position;
a driving member being connected to the transmission member in order to drive the transmission member to move between the opening position and the closing position.

2. The pneumatic electrical switch according to claim 1, wherein there are a plurality of air passages, a plurality of valve cores being correspondingly matched with the plurality of air passages, and a plurality of transmission members being installed and correspondingly matched with the plurality of valve cores respectively.

3. The pneumatic electrical switch according to claim 1, wherein the connecting member comprises a connecting member body installing a mounting hole penetrating being movably matched with the transmission member through the connecting member body,
a first connecting arm being installed on the connecting member body and connected to the pneumatic valve component,
and a second connecting arm being installed on the connecting member body and connected to the pneumatic valve component, wherein the second connecting arm is separated from the first connecting arm.

4. The pneumatic electrical switch according to claim 3, wherein the connecting member body, the first connecting arm and the second connecting arm are formed integrally.

5. The pneumatic electrical switch according to claim 3, wherein
the first connecting arm is connected to the first flank of the valve body, the second connecting arm is connected to the second flank of the valve body. Thereof the first flank is opposite to the second flank.

6. The pneumatic electrical switch according to claim 3, wherein the connecting member body sets a through hole penetrating through the connecting member body along the thickness direction of the connecting member body.

7. The pneumatic electrical switch according to claim 1, wherein the driving member comprises
a fixing portion being installed on the connecting member,
a driving member body being rotatable installed on the fixing portion and a groove being installed at the end surface of the driving member body; one side wall of the groove being installed in an inclined way, and thereof the driving member being contacted with the bottom wall of the groove at the closing position, and the driving member being contacted with the end face of the driving member body at the opening position,
and a holding portion being installed on the driving member body.

8. The pneumatic electrical switch according to claim 7, wherein a clamping trough being installed inside the fixing portion and a part of the connecting member is matched with the clamping trough.

9. A medical equipment, wherein comprising the pneumatic electrical switch according to any one of claims 1 to 8.

10. The medical equipment according to claim 9, wherein the medical equipment is a ventilator or an anesthesia machine.
